Europäisches Patentamt

European Patent Office

Office européen des brevets

(11) Veröffentlichungsnummer: **0 288 741**

**A2**

# (12) EUROPÄISCHE PATENTANMELDUNG

(21) Anmeldenummer: 88104705.4

(22) Anmeldetag: 24.03.88

(51) Int. Cl.⁴: **A45D 34/04 , A45D 40/26**

(30) Priorität: 11.04.87 DE 8705427 U

(43) Veröffentlichungstag der Anmeldung:
**02.11.88 Patentblatt 88/44**

(84) Benannte Vertragsstaaten:
**AT BE CH DE ES FR GB GR IT LI LU NL SE**

(71) Anmelder: **Riezler, Margret**
**Eggstrasse 2**
**A-8964 Riezlern(AT)**

(72) Erfinder: **Riezler, Margret**
**Eggstrasse 2**
**A-8964 Riezlern(AT)**

(74) Vertreter: **Hutzelmann, Gerhard**
**Duracher Strasse 22**
**D-8960 Kempten(DE)**

(54) **Pflegemaske.**

(57) Gesichtspflegemaske mit einer dem Gesicht angepaßten Außenlage aus elastischem Material, an der Befestigungsbänder angebracht sind. Das eigentliche Pflegematerial ist auf diese Außenlage aufgebracht und vorzugsweise durch eine durchlässige Innenlage abgedeckt.

Fig, 1

EP 0 288 741 A2

# Pflegemaske

Die Erfindung betrifft eine Pflegemaske, insbesondere eine Gesichtspflegemaske, mit einer der Form des zu pflegenden Körperteils angepaßten bzw. sich anpassenden Außenlage aus einem elastischen Material, wie z.B. einer Kunststoffolie.

Im Bedarfsfall herzustellende und dann aufzutragende Pflegemasken, insbesondere für das Gesicht, haben sich seit langem bewährt.

Der Erfindung liegt die Aufgabe zugrunde, eine Pflegemaske vorzuschlagen, die fertig zubereitet aufbewahrt werden kann und im Bedarfsfall schnell einsetzbar ist.

Diese Aufgabe wird erfindungsgemäß dadurch gelöst, daß die Außenlage als Abdeckung für das eigentliche Pflegematerial dient und mit Befestigungsmitteln, wie z.B. Bändern versehen ist.

Durch diese Ausgestaltung der Pflegemaske mit einer Außenlage, die sowohl als Träger als auch als Abdeckung für das Pflegematerial dient, kann die Pflegemaske konfektioniert und fertigt verkauft werden. Je nach Zusammensetzung des Pflegematerials ist eventuell eine Kühlung oder sogar ein Einfrieren notwendig; bei der erfindungsgemäßen Ausgestaltung ist dies ohne weiteres möglich.

Bei einer sehr vorteilhaften Ausgestaltung der Erfindung als Gesichtspflegemaske ist vorgesehen, daß im Bereich der Augen Öffnungen in der Außenlage vorgesehen sind.

Dadurch ist bei der Anwendung der Gesichtspflegemaske keinerlei Behinderung gegeben.

Zur zusätzlichen Pflege der Augen kann, gemäß einer weiteren Ausgestaltung der Erfindung, vorgesehen sein, daß für die Augen aus der eigentlichen Pflegemaske heraustrennbare oder getrennt hergestellte Pflege-und/oder Beruhigungskissen vorgesehen sind.

Sehr vorteilhaft kann es auch sein, wenn erfindungsgemäß das Pflegematerial zwischen der Außenlage und einer durchlässigen Innenlage eingebettet ist.

Die Wirksamkeit des Pflegematerials wird durch diese durchlässige Innenlage nicht wesentlich beeinflußt, sie ergibt aber eine vereinfachte Anwendbarkeit der Pflegemaske, da diese ohne Hinterlassen nennenswerter Rückstände jederzeit abgenommen werden kann. So kann also bei aktuellem Anlaß die Einwirkung der Pflegemaske unterbrochen und beliebig fortgesetzt werden.

Sehr vorteilhaft ist es auch, wenn erfindungsgemäß an den Öffnungs-Rändern im Bereich der Augen die Außenlage mit der Innenlage verschweißt ist.

Dadurch wird wirksam verhindert, daß das Pflegematerial an dieser Stelle zwischen den beiden Lagen hervortritt und in die Augen gelangt.

Weiterhin kann es sinnvoll sein, wenn erfindungsgemäß die Außenlage und die Innenlage am Außenumfang miteinander verschweißt sind.

Auch hierdurch wird ein Austreten des Pflegematerials und damit eine Verschmutzung der Kleider verhindert.

Als sehr vorteilhaft hat sich auch erwiesen, wenn erfindungsgemäß wenigstens am Außenumfang eine Rille od.dgl. eingeprägt ist

Diese Rille od.dgl. versteift die Pflegemaske so weit, daß sie einen einwandfreien Sitz aufweist und nicht unkontrolliert absteht.

Eine ähnliche Versteifungs-und Stabilisierungswirkung wird erfindungsgemäß auch dadurch erzielt, daß wenigstens am Außenumfang ein Versteifungswulst angeordnet ist.

Versteifungs-Rille bzw. Versteifungs-Wulst können auch vorteilhaft am Umfangs-Rand der Augenöffnungen vorgesehen sein.

In der Zeichnung ist die Erfindung anhand eines Ausführungsbeispieles veranschaulicht. Dabei zeigen:

Fig. 1 eine Außenansicht einer Pflegemaske mit Öffnungen im Bereich der Augen und mit seitlichen Befestigungsbändern,

Fig. 2 einen Querschnitt durch eine Pflegemaske mit Außenlage und einer Innenlage,

Fig. 3 eine Ansicht eines ellipsenförmig ausgebildeten Pflegekissens für die Augen,

Fig. 4 eine Ansicht eines ringförmig ausgebildeten Pflegekissens,

Fig. 5 eine Ansicht einer weiteren Pflegemaske mit eingeprägter Rille,

Fig. 6 einen Querschnitt durch die Pflegemaske gemäß Fig.5 und

Fig. 7 einen Querschnitt durch eine ähnliche Pflegemaske mit einem Wulst.

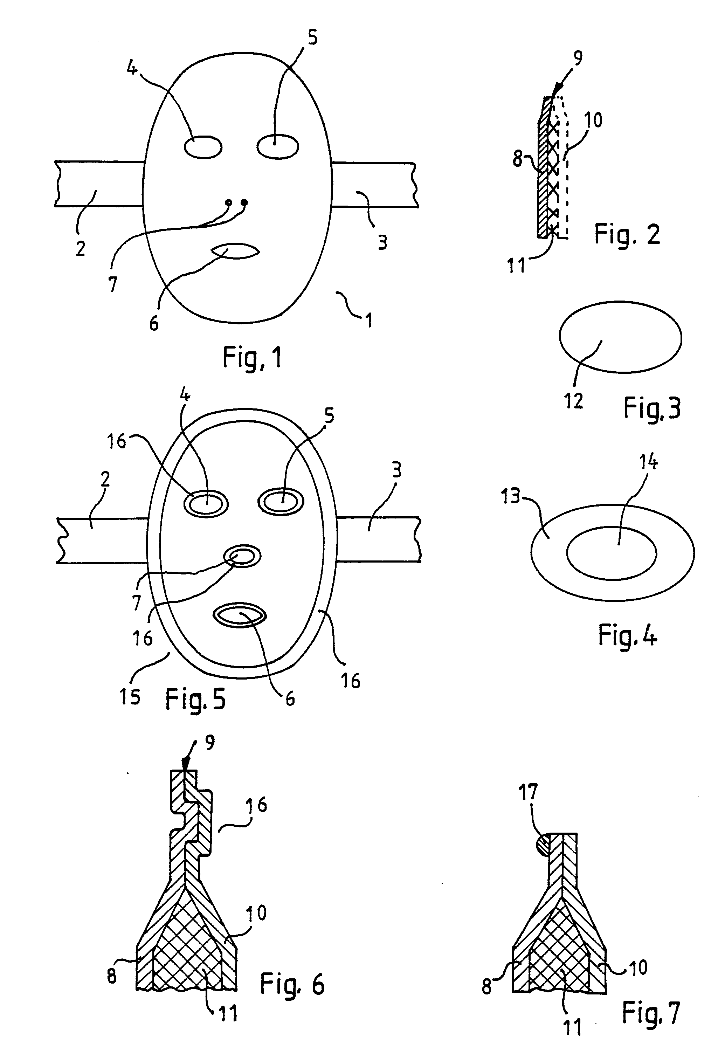

Mit 1 ist in Fig.1 eine Pflegemaske bezeichnet, die in nicht näher dargestellter Weise der Gesichtsform eines Menschen angepaßt ist. An den beiden Seitenwänden der Pflegemaske 1 ist je ein Band 2,3 als Befestigungsmittel vorgesehen. Die nicht dargestellten freien Enden dieser Befestigungsbänder können beispielsweise hinter dem Kopf zusammengebunden werden, um so der Pflegemaske einen ausreichenden Halt zu geben. Es sind jedoch auch weitere Hilfsmittel zum Verbinden der beiden Bänder 2,3, wie z.B. Klettverschlüsse, Haken und Ösen usw. denkbar.

In der eigentlichen Pflegemaske 1 sind zwei Öffnungen4,5 für die Augen, eine weitere Öffnung 6 für den Mund und eine Doppelöffnung 7 zum Atmen durch die Nase vorgesehen.

In Fig.2 ist an einem Abschnitt der Aufbau der Pflegemaske verdeutlicht.

Als Träger ist eine Außenlage 8 vorgesehen, die aus einer Kunststoffolie besteht; diese ist am Rand 9 mit einer Innenlage 10 verbunden, die durchlässig ist und beispielsweise aus Gaze besteht. Zwischen Außenlage 8 und Innenlage 10 ist das eigentliche Pflegematerial 11 eingebracht, das seine Wirkung durch die Innenlage hindurch ausübt. Je nach der Art und der Konsistenz dieses Pflegematerials muß die Innenlage beschaffen sein. So ist neben einer Gaze auch der Einsatz einer Gitterfolie, eines Fadengeleges oder eines Gewebes möglich und zweckmäßig.

In Fig.3 ist ein Pflegekissen 12 dargestellt, das der Form eines Auges angepaßt ist und aus der Pflegemaske 1 im Bereich der Öffnungen 4 bzw. 5 herausgetrennt ist. Der Aufbau des Pflegekissens entspricht dabei völlig dem der Pflegemaske und ist für die getrennte Auflage auf die Augen vorgesehen. Es ist aber auch möglich, ein solches Pflegekissen 12 getrennt von der Pflegemaske herzustellen, wodurch sein Aufbau unabhängig von dieser ist und andere Pflegebestandteile enthalten kann.

Ein ringförmig ausgebildetes Pflegekissen 13 ist in Fig.4 dargestellt, welches das Öffnen des Auges während der Pflegebehandlung ermöglicht, da eine Durchblicköffnung 14 vorgesehen ist.

Beim Ausführungsbeispiel nach Fig.5 ist eine Pflegemaske 15 gezeigt, die an ihren Rändern jeweils mit einer Rille 16 versehen ist, wie dies in Fig.6 näher dargestellt wurde. Es handelt sich dabei um eine Ausführung mit Außenlage 8 und Innenlage 10, die beide am Rand 9 miteinander verschweißt sind, wobei das Pflegematerial 11 zwischen beiden Lagen eingeschlossen ist. Im Bereich des Randes 9 ist dann die Rille 16 in beide Lagen geprägt, wodurch eine zusätzliche Versteifung erzielt ist.

Beim Ausführungsbeispiel nach Fig.7 ist anstelle der Rille 16 ein Versteifungs-Wulst 17 an der Außenlage 8 angeordnet.

Sowohl die Rille 16 als auch der Wulst 17 können an allen Rändern der Pflegemaske angeordnet sein, wie dies in Fig.5 dargestellt wurde.

## Ansprüche

1. Pflegemaske, insbesondere Gesichtspflegemaske, mit einer der Form des zu pflegenden Körperteils angepaßten bzw. sich anpassenden Außenlage aus einem elastischen Material, wie z.B. einer Kunststoffolie, **dadurch gekennzeichnet**, daß die Außenlage(8) als Träger und/oder als Abdeckung für das eigentliche Pflegematerial(11) dient und mit Befestigungsmitteln, wie z.B. Bändern(2,3) versehen ist.

2. Pflegemaske nach Anspruch 1 zur Verwendung als Gesichtspflegemaske, **dadurch gekennzeichnet**, daß im Bereich der Augen Öffnungen-(4,5) in der Außenlage(8) vorgesehen sind.

3. Pflegemaske nach Anspruch 2, **dadurch gekennzeichnet**, daß für die Augen aus der eigentlichen Pflegemaske heraustrennbare oder getrennt hergestellte Pflege-und/oder Beruhigungskissen(12,13) vorgesehen sind.

4. Pflegemaske nach einem der vorhergehenden Ansprüche, **dadurch gekennzeichnet**, daß das Pflegematerial(11) zwischen der Außenlage(8) und einer durchlässigen Innenlage(10) eingebettet ist.

5. Pflegemaske nach Anspruch 4, **dadurch gekennzeichnet**, daß an den Öffnungs-Rändern im Bereich der Augen die Außenlage(8) mit der Innenlage(10) verschweißt ist.

6. Pflegemaske nach Anspruch 4 od. 5, **dadurch gekennzeichnet**, daß die Außenlage(8) und die Innenlage(10) am Außenumfang miteinander verschweißt sind.

7. Pflegemaske nach einem der vorhergehenden Ansprüche, **dadurch gekennzeichnet**, daß wenigstens am Außenumfang eine Rille(16) od.dgl. eingeprägt ist.

8. Pflegemaske nach einem der Ansprüche 1 bis 6, **dadurch gekennzeichnet**, daß wenigstens am Außenumfang ein Versteifungswulst(17) angeordnet ist.

Fig. 1

Fig. 2

Fig. 3

Fig. 4

Fig. 5

Fig. 6

Fig. 7